# EUROPÄISCHE PATENTSCHRIFT

(11) **EP 2 408 764 B1**
(45) Veröffentlichungstag und Bekanntmachung des Hinweises auf die Patenterteilung: **17.07.2013**
(21) Anmeldenummer: 10708509.4
(22) Anmeldetag: 12.03.2010
(51) Int. Cl.: C07D 405/12, C07D 307/68

(54) **NEUES VERFAHREN ZUR HERSTELLUNG VON ENAMINOCARBONYL-VERBINDUNGEN**
New method for producing enaminocarbonyl compounds
Nouveau procédé de fabrication de composés d'énaminocarbonyles

(30) Priorität: 16.03.2009 EP 09155199
(43) Veröffentlichungstag der Anmeldung: 25.01.2012
(73) Patentinhaber: Bayer CropScience AG, 40789 Monheim (DE)
(72) Erfinder: LUI, Norbert, 51519 Odenthal (DE); HEINRICH, Jens-Dietmar, 51399 Burscheid (DE)
(86) Internationale Anmeldenummer: PCT/EP2010/001568
(87) Internationale Veröffentlichungsnummer: WO 2010/105772

(56) Entgegenhaltungen:
- WO-A-2007/115644
- HANS-DIETRICH STACHEL ET AL: "DIE SYNTHESE VON PIPEROLID UND VERWANDTEN TETRONSÄUREN" TETRAHEDRON LETTERS, ELSEVIER, AMSTERDAM, NL LNKD- DOI:10.1016/S0040-4039(00)78636-9, Bd. 21, 1. Januar 1980 (1980-01-01), Seiten 2891-2892, XP002529087 ISSN: 0040-4039

## Beschreibung

Die vorliegende Erfindung betrifft ein Verfahren zur Herstellung von Enaminocarbonyl-Verbindungen.

Bestimmte substituierte Enaminocarbonyl-Verbindungen sind als insektizid wirksame Verbindungen aus der EP 0 539 588 A1 bekannt. Darüber hinaus beschreiben auch die internationalen Patentanmeldungen WO 2007/115644, WO 2007/115643 und WO 2007/115646 entsprechende insektizid wirksame Enaminocarbonyl-Verbindungen.

Im Allgemeinen werden Enaminocarbonyl-Verbindungen aus Tetronsäure und einem Amin gemäß dem nachfolgenden Schema 1 synthetisiert. Diese Vorgehensweise ist beispielsweise in EP 0 539 588 A1 sowie in Heterocycles Vol. 27, Nr. 8, Seite 1907 bis 1923 (1988) beschrieben.

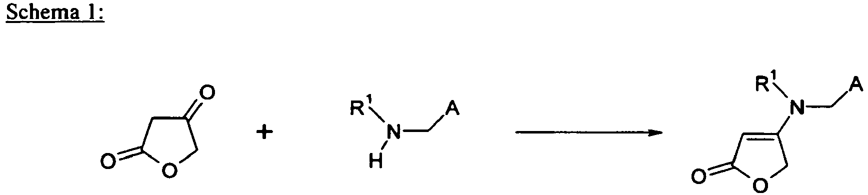

Nachteilig an diesem Verfahren ist insbesondere, dass man wasserfreie Tetronsäure als Ausgangsverbindung benötigt, deren Herstellung aufwändig und kostenintensiv ist.

So wird Tetronsäure im Allgemeinen ausgehend von Acetessigester über eine Bromierung und anschließende Hydrierung hergestellt (vgl. Synthetic Communication, 11(5), Seiten 385 bis 390 (1981)). Die Gesamtausbeute an Tetronsäure ausgehend von Acetessigester ist dabei kleiner als 40 %, was das Verfahren unter industriellem Gesichtspunkt wenig attraktiv macht.

In der CH-PS 503 722 ist ein weiteres Verfahren zur Herstellung von Tetronsäure beschrieben. Dabei wird 4-Chloracetessigester mit einem aromatischen Amin zum 3-Arylaminocrotonlacton umgesetzt und anschließend wird die Tetronsäure durch Behandlung mit Mineralsäuren freigesetzt. Der Nachteil an diesem Verfahren besteht darin, dass die Isolierung der Tetronsäure nur durch Hochvakuum-Sublimation möglich ist, was auch dieses Verfahren unter industriellem Gesichtspunkt wenig attraktiv macht.

Ein weiteres Verfahren zur Herstellung von Tetronsäure ist in der EP 0 153 615 A beschrieben, in welchem von 2,4-Dichloracetessigestern ausgegangen wird. Dieses ebenfalls mehrstufige und aufwändige Verfahren liefert die gewünschte Verbindung ebenfalls nur mit einer mäßigen Gesamtausbeute von 65 %.

In Tetrahedron Letters, Nr. 31, Seiten 2683 und 2684 (1974) ist die Herstellung von Tetronsäure und einer entsprechenden Enaminocarbonyl-Verbindung beschrieben. Die dort beschriebene Synthese ist in folgendem Schema 2 wiedergegeben. Als Edukt wird dabei Acetylendicarbonsäuredimethylester eingesetzt.

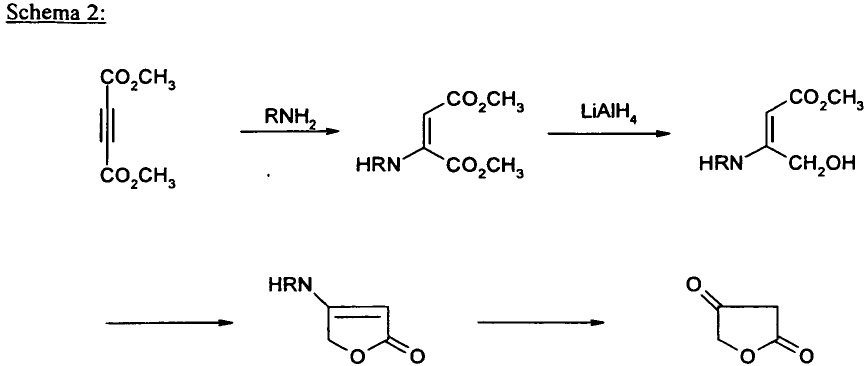

Nachteilig an diesem Verfahren ist die geringe Gesamtausbeute von nur 30 % sowie das Erfordernis, kostenintensive Edukte, beispielsweise Lithiumaluminiumhydrid (LiAlH₄), als Reagenzien verwenden zu müssen.

Ferner ist aus dem Stand der Technik ein Verfahren zur Herstellung von Enaminocarbonyl-Verbindungen ausgehend von Methyltetronat bekannt (J. Heterocyclic Chem., 21, 1753 (1984)). Für dieses Verfahren wird als Ausgangsmaterial der kostenintensive 4-Brom-3-methoxy-but-3-encarbonsäureester verwendet.

Ein weiteres Verfahren geht von einem 4-Chloracetessigester aus, der mit Aminen umgesetzt wird (Heterocycles, Vol. 27, Nr. 8, 1988, Seiten 1907 bis 1923). Die Reaktion zum Aminofuran wird in einem Schritt durchgeführt. Dabei wird das Amin mit Eisessig zu einer Lösung von 4-Chloracetessigester in Benzol gegeben und die resultierende Mischung wird mehrere Stunden unter Rückfluss erhitzt. Die Ausbeuten an 4-Methylamino-2(5H)-furanon in dieser Synthese betragen nur 40 %.

Aus EP 0 123 095 A ist ein Verfahren bekannt, in welchem Tetronsäureamid aus 3-Amino-4-acetoxycrotonsäureester hergestellt wird. 3-Amino-4-acetoxycrotonsäureester ist kostenintensiv und aufwändig herzustellen, so dass eine wirtschaftliche Synthese mit diesem Verfahren nicht möglich ist.

Ein weiteres Verfahren zur Herstellung von Tetronsäure ausgehend von Malonestern und Chloracetylchlorid ist aus J. Chem. Soc., Perkin Trans. 1 (1972), Nr. 9/10, Seiten 1225 bis 1231 bekannt. Dieses Verfahren liefert die gewünschte Zielverbindung mit einer Ausbeute von nur 43 %.

In WO 2007/115644 wird die Herstellung von Enaminocarbonyl-Verbindungen, beispielsweise von 4-[[(6-Chlorpyridin-3-yl)methyl](3,3-dichlorprop-2-en-1-yl)amino]furan-2(5H)-on durch Umsetzung von 4-[[(6-Chlorpyridin-3-yl)methyl]amino]furan-2(5H)-on mit 3-Brom-1,1-dichlorprop-1-en, beschrieben (vgl. Herstellungsbeispiel, Verfahren 2, Beispiel (3)). WO 2007/115644 beschreibt auch die Herstellung von Enaminocarbonyl-Verbindungen, beispielsweise von 4-[[(6-Chlorpyridin-3-yl)methyl](2-Fluorethyl)amino]furan-2(5H)-on durch Umsetzung von 4-[[(2-Fluorethyl)amino]furan-2(5H)-on mit 2-Chlor-5-chlormethyl-pyridin (vgl. Herstellungsbeispiele, Verfahren 3, Beispiel (4)). Die Reaktionen werden vorzugsweise mit Hydriden des Lithiums oder Natriums durchgeführt. Diese Substrate sind im Allgemein kostenintensiv und gleichzeitig aus Sicherheitsgründen nur schwer zu handhaben.

In WO 2009/036899, die die Priorität der Europäischen Patentanmeldung Nr. 07116639 beansprucht, werden Enaminocarbonylverbindungen beispielsweise ausgehend von 4-(Methoxycarbonyl)-5-oxo-2,5-dihydrofuran-3-ol und einem Amin hergestellt. 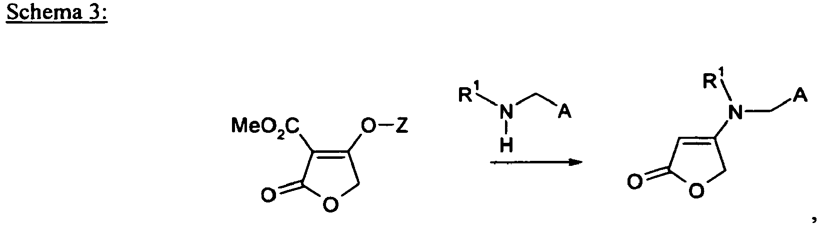 wobei
- R¹: für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl oder Arylalkyl steht;
- Z: für Wasserstoff, Alkalimetall oder Erdalkalimetall steht; und
- A: Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder für
Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl steht, welcher gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für 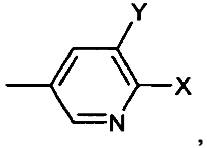 in welchem
- X: für Halogen, Alkyl oder Halogenalkyl steht und
- Y: für Halogen, Alkyl, Halogenalkyl, Halogenalkoxy, Azido oder Cyan steht.

Ausgehend von diesem Stand der Technik ergibt sich als Aufgabe der vorliegenden Erfindung, ein alternatives Verfahren zur Herstellung von Enaminocarbonyl-Verbindungen bereitzustellen, welches vorzugsweise einfach und kostengünstig durchzuführen ist. Die mit diesem angestrebten Verfahren erhältlichen Enaminocarbonyl-Verbindungen sollen dabei vorzugsweise mit hoher Ausbeute und hoher Reinheit erhalten werden. Insbesondere soll das angestrebte Verfahren den Erhalt der gewünschten Zielverbindungen ohne die Notwendigkeit komplexer Aufreinigungsmethoden ermöglichen.

Gelöst wird diese Aufgabe durch ein neues Verfahren zur Herstellung von Enaminocarbonyl-Verbindungen der allgemeinen Formel (I): 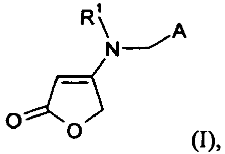 wobei das erfindungsgemäße Verfahren dadurch gekennzeichnet ist, dass Verbindungen der allgemeinen Formel (II) 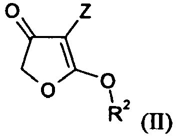 mit Aminen der allgemeinen Formel (III) 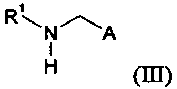 umgesetzt werden, wobei die einzelnen Reste folgende Bedeutung aufweisen:
- R¹: steht für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl oder Arylalkyl;
- R²: steht für Alkyl, Halogenalkyl, Alkenyl, Halogenalkenyl, Alkinyl, Cycloalkyl, Cycloalkylalkyl, Halogencycloalkyl, Alkyloxyalkyl, Halogencycloalkylalkyl, Aryl oder Arylalkyl;
- Z: ist ausgewählt aus (C=O)OR³ und (C=O)NR¹CH₂A, wobei R³ ausgewählt ist aus C₁₋₁₂-Alkyl-, C₃₋₈-Cycloalkyl, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen substituiert sein können, die ausgewählt sind aus der Gruppe bestehend aus -R', -OR', -SR', -NR'₂, -SiR'₃, -COOR', -(C=O)R', -CN und -CON'R₂', wobei R' Wasserstoff oder eine C₁₋₁₂-AlkylGruppe ist;
- A: steht für Pyrid-2-yl oder Pyrid-4-yl oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlor-pyrazin-5-yl oder für 1,3-Thiazol-5-yl, welches gegebenenfalls in 2-Position substituiert ist durch Chlor oder Methyl, oder
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welches gegebenenfalls durch Fluor, Chlor, Brom, Cyano, Nitro, C₁-C₄-Alkyl (welches gegebenenfalls durch Fluor und/oder Chlor substituiert ist), C₁-C₃-Alkylthio (welches gegebenenfalls durch Fluor und/oder Chlor substituiert ist), oder C₁-C₃-Alkylsulfonyl (welches gegebenenfalls durch Fluor und/oder Chlor substituiert ist), substituiert ist, oder für

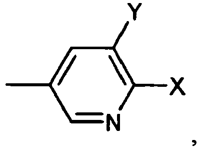

wobei
- X: für Halogen, Alkyl oder Halogenalkyl steht und
- Y: für Halogen, Alkyl, Halogenalkyl, Halogenalkoxy, Azido oder Cyan steht.

Erfindungsgemäß ist somit vorgesehen, dass die gewünschten Enaminocarbonyl-Verbindungen der Formel (I) durch eine Umsetzung der entsprechenden Verbindungen der Formel (II) mit Aminen der Formel (III) hergestellt werden. Die gewünschten Enaminocarbonyl-Verbindungen der Formel (I) werden unter den weiter unten näher spezifizierten Reaktionsbedingungen mit guten Ausbeuten in hoher Reinheit erhalten, womit das erfindungsgemäße Verfahren die Nachteile der bekannten Verfahren überwindet. Die gewünschten Verbindungen werden dabei in einer Reinheit erhalten, welche eine umfangreiche Aufarbeitung des unmittelbaren Reaktionsprodukts im Allgemeinen nicht erforderlich macht.

Bevorzugte, besonders bevorzugte und ganz besonders bevorzugte Bedeutungen der in den oben erwähnten allgemeinen Formeln (I) und (III) aufgeführten Reste A und R¹ werden im Folgenden erläutert.
- A: wird bevorzugt ausgewählt aus der Gruppe bestehend aus 6-Fluor-pyrid-3-yl, 6-Chlorpyrid-3-yl, 6-Brom-pyrid-3-yl, 6-Methyl-pyrid-3-yl, 6-Trifluormethyl-pyrid-3-yl, 6-Trifluormethoxypyrid-3-yl, 6-Chlor-1,4-pyridazin-3-yl, 6-Methyl-1,4-pyridazin-3-yl, 2-Chlor-1,3-thiazol-5-yl oder 2-Methyl-1,3-thiazol-5-yl, 2-Chlor-pyrimidin-5-yl, 2-Trifluormethyl-pyrimidin-5-yl, 5,6-Difluor-pyrid-3-yl, 5-Chlor-6-fluor-pyrid-3-yl, 5-Brom-6-fluor-pyrid-3-yl, 5-Iod-6-fluor-pyrid-3-yl, 5-Fluor-6-chlor-pyrid-3-yl, 5,6-dichlor-pyrid-3-yl, 5-Brom-6-chlor-pyrid-3-yl, 5-Iod-6-chlor-pyrid-3-yl, 5-Fluor-6-brom-pyrid-3-yl, 5-Chlor-6-brom-pyrid-3-yl, 5,6-Dibrom-pyrid-3-yl, 5-Fluor-6-iod-pyrid-3-yl, 5-Chlor-6-iod-pyrid-3-yl, 5-Brom-6-iod-pyrid-3-yl, 5-Methyl-6-fluor-pyrid-3-yl, 5-Methyl-6-chlorpyrid-3-yl, 5-Methyl-6-brom-pyrid-3-yl, 5-Methyl-6-iod-pyrid-3-yl, 5-Difluormethyl-6-fluor-pyrid-3-yl, 5-Difluormethyl-6-chlor-pyrid-3-yl, 5-Difluormethyl-6-brom-pyrid-3-yl und 5-Difluormethyl-6-iod-pyrid-3-yl.
- R¹: wird bevorzugt ausgewählt aus der Gruppe bestehend aus Wasserstoff, Alkyl, Halogenalkyl, Alkenyl, Halogenalkenyl, Alkinyl, Cycloalkyl, Cycloalkylalkyl, Halogencycloalkyl, Halogencycloalkylalkyl und Alkoxyalkyl.
- A: wird besonders bevorzugt ausgewählt aus der Gruppe bestehend aus 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlor-pyrid-3-yl, 5,6-Dichlor-pyrid-3-yl, 5-Brom-6-chlor-pyrid-3-yl, 5-Fluor-6-brom-pyrid-3-yl, 5-Chlor-6-brom-pyrid-3-yl, 5,6-Dibrom-pyrid-3-yl, 5-Methyl-6-chlor-pyrid-3-yl, 5-Chlor-6-iod-pyrid-3-yl und 5-Difluormethyl-6-chlorpyrid-3-yl.
- R¹: wird besonders bevorzugt ausgewählt aus der Gruppe bestehend aus Methyl, Ethyl, n-Propyl, iso-Propyl , Vinyl, Allyl, Propargyl, Cyclopropyl, Alkoxyalkyl, 2-Fluor-ethyl, 2,2-Difluor-ethyl und 2-Fluor-cyclopropyl.
- A: wird ganz besonders bevorzugt ausgewählt aus der Gruppe bestehend aus 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl und 5-Fluor-6-brom-pyrid-3-yl.
- R¹: wird ganz besonders bevorzugt ausgewählt aus der Gruppe bestehend aus Methyl, Ethyl, n-Propyl, iso-Propyl, n-Prop-2-enyl, n-Prop-2-inyl, Cyclopropyl, Methoxyethyl, 2-Fluorethyl und 2,2-Difluor-ethyl.

Bevorzugte, besonders bevorzugte und ganz besonders bevorzugte Bedeutungen der in der oben erwähnten allgemeinen Formel (II) aufgeführten Reste Z und R² werden im Folgenden erläutert.
- Z: wird bevorzugt ausgewählt aus der Gruppe bestehend aus (C=O)OR³ , wobei R³ ausgewählt ist aus C₁₋₁₂-Alkyl-, C₃₋₈-Cycloalkyl-, C₂₋₁₂-Alkenyl-, C₂₋₁₂-Alkinyl-, C₆₋₈-Aryl-, C₇₋₁₉-Arylalkyl- oder C₇₋₁₉-Alkylaryl-Gruppen, die jeweils mit einer oder mehreren Gruppen substituiert sein können, die ausgewählt sind aus der Gruppe bestehend aus -R', -OR', -SR', wobei R' Wasserstoff oder eine C₁₋₁₂-Alkyl-Gruppe ist;
- R²: wird bevorzugt ausgewählt aus der Gruppe bestehend aus Alkyl, Halogenalkyl, Alkenyl, Halogenalkenyl, Cycloalkyl, Alkyloxyalkyl oder Arylalkyl.
- Z: wird besonders bevorzugt ausgewählt aus der Gruppe bestehend aus (C=O)OR³, wobei R³ ausgewählt ist aus Methyl, Ethyl, n-Propyl, iso-Propyl oder Butyl;
- R²: wird besonders bevorzugt ausgewählt aus der Gruppe bestehend aus C₁-C₁₂-Alkyl.
- Z: wird ganz besonders bevorzugt ausgewählt aus der Gruppe bestehend aus (C=O)OR³, wobei R³ ausgewählt ist aus Methyl und Ethyl;
- R²: wird ganz besonders bevorzugt ausgewählt aus der Gruppe bestehend aus Ethyl, Isopropyl und Butyl.

In einer bevorzugten Ausführungsform werden in dem erfindungsgemäßen Verfahren Ausgangsverbindungen der Formeln (II) und (III) eingesetzt, in welchen die Substituenten A, Z, R¹, R² und R³ die vorstehend erwähnten bevorzugten Bedeutungen aufweisen, wobei die bevorzugten, besonders bevorzugten und ganz bevorzugten Bedeutungen der Substituenten kombiniert werden können.

In einer besonders bevorzugten Ausführungsform der vorliegenden Erfindung werden in dem erfindungsgemäßen Verfahren Ausgangsverbindungen der allgemeinen Formeln (II) und (III) eingesetzt, in welchen die Substituenten A, Z, R¹, R² und R³ die vorstehend erwähnten besonders bevorzugten Bedeutungen aufweisen, wobei die bevorzugten, besonders bevorzugten und ganz bevorzugten Bedeutungen der Substituenten kombiniert werden können.

In einer ganz besonders bevorzugten Ausführungsform der vorliegenden Erfindung werden in dem erfindungsgemäßen Verfahren Ausgangsverbindungen der allgemeinen Formeln (II) und (III) eingesetzt, in welchen die Substituenten A, Z, R¹; R² und R³ die vorstehend erwähnten ganz besonders bevorzugten Bedeutungen aufweisen, wobei die bevorzugten, besonders bevorzugten und ganz bevorzugten Bedeutungen der Substituenten kombiniert werden können.

Sofern nichts anderes erwähnt wird unter dem Begriff "Alkyl", entweder in Alleinstellung oder aber in Kombination mit weiteren Begriffen, wie beispielsweise Halogenalkyl, Alkoxyalkyl, Cycloalkylalkyl, Halogencycloalkylalkyl und Arylalkyl, im Rahmen der vorliegenden Erfindung ein Rest einer gesättigten, aliphatischen Kohlenwasserstoffgruppe mit 1 bis 12 Kohlenstoffatomen verstanden, die verzweigt oder unverzweigt sein kann. Beispiele für C₁-C₁₂-Alkylreste sind Methyl, Ethyl, n-Propyl, iso-Propyl, n-Butyl, iso-Butyl, sek.-Butyl, tert.-Butyl, n-Pentyl, iso-Pentyl, Neopentyl, tert.-Pentyl, 1-Methylbutyl, 2-Methylbutyl, 1-Ethylpropyl, 1,2-Dimethylpropyl, Hexyl, n-Heptyl, n-Octyl, n-Nonyl, n-Decyl, n-Undecyl und n-Dodecyl. Von diesen Alkylresten sind C₁-C₆-Alkylreste besonders bevorzugt. Insbesondere bevorzugt sind C₁-C₄-Alkylreste, speziell Methyl und Ethyl.

Sofern nichts anderes erwähnt wird unter dem Begriff "Alkenyl", entweder in Alleinstellung oder aber in Kombination mit weiteren Begriffen, erfindungsgemäß ein linearer oder verzweigter C₂-C₁₂-Alkenylrest, welcher mindestens eine Doppelbindung aufweist, beispielsweise Vinyl, Allyl, 1-Propenyl, Isopropenyl, 1-Butenyl, 2-Butenyl, 3-Butenyl, 1,3-Butadienyl, 1-Pentenyl, 2-Pentenyl, 3-Pentenyl, 4-Pentenyl, 1,3-Pentadienyl, 1-Hexenyl, 2-Hexenyl, 3-Hexenyl, 4-Hexenyl, 5-Hexenyl und 1,4-Hexadienyl, verstanden. Bevorzugt hiervon sind C₂-C₆-Alkenylreste und besonders bevorzugt sind C₂-C₄-Alkenylreste.

Sofern nichts anderes erwähnt wird unter dem Begriff "Alkinyl", entweder in Alleinstellung oder aber in Kombination mit weiteren Begriffen, erfindungsgemäß ein linearer oder verzweigter C₂-C₁₂-Alkinylrest, welcher mindestens eine Dreifachbindung aufweist, beispielsweise Ethinyl, 1-Propinyl und Propargyl, verstanden. Bevorzugt hiervon sind C₃-C₆-Alkinylreste und besonders bevorzugt sind C₃-C₄-Alkinylreste. Der Alkinylrest kann dabei auch mindestens eine Doppelbindung aufweisen.

Sofern nichts anderes erwähnt wird unter dem Begriff "Cycloalkyl", entweder in Alleinstellung oder aber in Kombination mit weiteren Begriffen, erfindungsgemäß ein C₃-C₈-Cycloalkylrest verstanden, beispielsweise Cyclopropyl, Cyclobutyl, Cyclopentyl, Cyclohexyl, Cycloheptyl und Cyclooctyl, verstanden. Bevorzugt hiervon sind C₃-C₆-Cycloalkylreste.

Sofern nichts anderes erwähnt wird unter dem Begriff "Aryl" erfindungsgemäß ein aromatischer Rest mit 6 bis 14 Kohlenstoffatomen, vorzugsweise Phenyl, verstanden.

Sofern nichts anderes erwähnt wird unter dem Begriff "Arylalkyl" eine Kombination von erfindungsgemäß definierten Resten "Aryl" und "Alkyl" verstanden, wobei der Rest im Allgemeinen über die Alkylgruppe gebunden wird. Beispiele hierfür sind Benzyl, Phenylethyl oder α-Methylbenzyl, wobei Benzyl besonders bevorzugt ist.

Sofern nichts anderes erwähnt werden unter dem Begriff "durch Halogen substituierte Reste" beispielsweise Halogenalkyl, einfach oder mehrfach bis zur maximal möglichen Substituentenzahl halogenierte Reste verstanden. Bei mehrfacher Halogenierung können die Halogenatome gleich oder verschieden sein. Halogen steht dabei für Fluor, Chlor, Brom oder Iod, insbesondere für Fluor, Chlor oder Brom.

Sofern nichts anderes erwähnt wird unter dem Begriff "Alkoxy", entweder in Alleinstellung oder aber in Kombination mit weiteren Begriffen, wie beispielsweise Halogenalkoxy, vorliegend ein Rest O-Alkyl verstanden, wobei der Begriff "Alkyl" die oben stehende Bedeutung aufweist.

Gegebenenfalls substituierte Reste können einfach oder mehrfach substituiert sein, wobei bei einer Mehrfachsubstitution die Substituenten gleich oder verschieden sein können.

Die Verbindungen der allgemeinen Formel (II) sind partiell aus dem Stand der Technik bekannt (R. Anschütz, Chemische Berichte, 1912, 45, 2374; E. Benary, Chemische Berichte, 1912, 45, 3682; Kuo, Sheng Chut et al., Journal of Heterocyclic Chemistry, 1989, 26(3), 605-8) oder können analog hergestellt werden.

Ein weiterer Gegenstand der vorliegenden Erfindung sind Verbindungen der Formel (II) 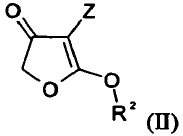 in welcher die Reste Z und R² wie oben definiert sind, mit der Maßgabe, dass R² nicht für Ethyl oder Phenyl steht.

Gegenstand der Erfindung ist auch ein Verfahren zur Synthese entsprechender abgewandelter Derivate der Verbindungen der allgemeinen Formel (II) gemäß nachfolgendem Schema 4 beispielsweise ausgehend von Malonsäurederivaten der allgemeinen Formel (IV) unter Verwendung von Chloressigsäurechlorid in Gegenwart einer Base: 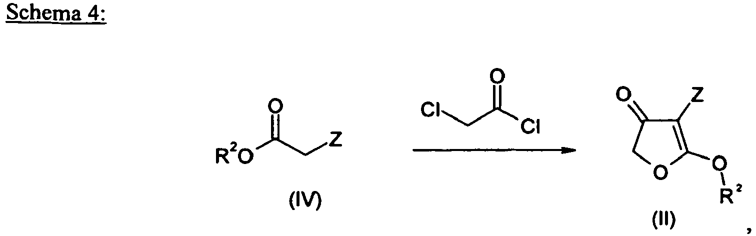 wobei Z und R² wie oben definiert sind.

Die als Edukte verwendeten Malonsäureester der allgemeinen Formel (IV) sind kommerziell erhältlich oder können nach aus dem Stand der Technik bekannten Verfahren hergestellt werden.

Die für die erfindungsgemäße Umsetzung erforderlichen Amine der allgemeinen Formel (III) sind kommerziell erhältlich oder können nach literaturbekannten Verfahren hergestellt werden (vgl. z.B. S. Patai " The Chemistry of Amino Group", Interscience Publishers, New York, 1968).

Die Umsetzung der Verbindungen der Formel (II) mit den Aminen der Formel (III) kann in Gegenwart von Lösungsmitteln (Verdünnungsmitteln) durchgeführt werden. Lösungsmittel werden vorteilhaft in einer solchen Menge eingesetzt, dass das Reaktionsgemisch während des ganzen Verfahrens gut rührbar bleibt. Als Lösungsmittel zur Durchführung des erfindungsgemäßen Verfahrens kommen alle unter den Reaktionsbedingungen inerten organischen Lösungsmittel in Frage.

Als Beispiele sind zu nennen: Halogenkohlenwasserstoffe, insbesondere Chlorkohlenwasserstoffe, wie Tetrachlorethylen, Tetrachlorethan, Dichlorpropan, Methylenchlorid, Dichlorbutan, Chloroform, Tetrachlorkohlenstoff, Trichlorethan, Trichlorethylen, Pentachlorethan, Difluorbenzol, 1,2-Dichlorethan, Chlorbenzol, Brombenzol, Dichlorbenzol, Chlortoluol, Trichlorbenzol; Ether, wie Ethylpropylether, Methyl-tert-butylether, n-Butylether, Anisol, Phenetol, Cyclohexylmethylether, Dimethylether, Diethylether, Dimethylglycol, Diphenylether, Dipropylether, Diisopropylether, Di-n-butylether, Diisobutylether, Diisoamylether, Ethylenglycoldimethylether, Isopropylethylether, Methyl-tert-butylether, Tetrahydrofuran, Methyl-Tetrahydrofuran, Dioxan, Dichlordiethylether; Methyl-THF und Polyether des Ethylenoxids und/oder Propylenoxids; Nitrokohlenwasserstoffe wie Nitromethan, Nitroethan, Nitropropan, Nitrobenzol, Chlornitrobenzol, o-Nitrotoluol; Nitrile wie Acetonitril, Methylnitril, Propionitril, Butyronitril, Isobutyronitril, Benzonitril, Phenylnitril, m-Chlorbenzonitril sowie Verbindungen wie Tetrahydrothiophendioxid und Dimethylsulfoxid, Tetramethylensulfoxid, Dipropylsulfoxid, Benzylmethylsulfoxid, Diisobutylsulfoxid, Dibutylsulfoxid, Diisoamylsulfoxid; Sulfone wie Dimethyl-, Diethyl-, Dipropyl-, Dibutyl-, Diphenyl-, Dihexyl-, Methylethyl-, Ethylpropyl-, Ethylisobutyl- und Pentamethylensulfon; aliphatische, cycloaliphatische oder aromatische Kohlenwasserstoffe wie Pentan, n-Hexan, n-Heptan, n-Oktan, Nonan beispielsweise sogenannte White Spirits mit Komponenten mit Siedepunkten im Bereich beispielsweise von 40°C bis 250°C, Cymol, Benzinfraktionen innerhalb eines Siedeintervalles von 70°C bis 190°C, Cyclohexan, Methylcyclohexan, Petrolether, Ligroin, Octan, Benzol, Toluol, Xylol; Ester wie Methyl-, Ethyl-, Butyl-, Isobutylacetat, sowie Dimethyl-, Dibutyl-, Ethylencarbonat; Amide wie Hexamethylenphosphorsäuretriamid, Formamid, N,N-Dimethyl-acetamid, *N*-Methyl-formamid, *N,N*-Dimethyl-formamid, *N,N*-Dipropyl-formamid, NN-Dibutyl-formamid, *N*-Methyl-pyrrolidin, N-Methyl-caprolactam, 1,3-Dimethyl-3,4,5,6-tetrahydro-2(1H)-pyrimidin, Octylpyrrolidon, Octylcaprolactam, 1,3-Dimethyl-2-imidazolindion, N-Formyl-piperidin, N,N'-1,4-Diformylpiperazin; und aliphatische Alkohole, wie Methanol, Ethanol, n-Propanol und iso-Propanol und n-Butanol.

Die erfindungsgemäße Umsetzung wird vorzugsweise in einem Lösungsmittel durchgeführt, welches ausgewählt ist aus der Gruppe bestehend aus Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

Gegebenenfalls ist es in Abhängigkeit der Ausgangsverbindungen auch möglich, die Umsetzung in Substanz, d.h. ohne Zusatz an Lösungsmitteln, durchzuführen.

Die Reaktion kann auch in Gegenwart von Wasser durchgeführt werden.

Die Umsetzung der Verbindungen der Formel (II) mit den Aminen der Formel (III) wird vorzugsweise in Gegenwart einer Brønstedt-Säure durchgeführt.

Das molare Verhältnis der eingesetzten Brenstedt-Säure zu den Aminen der Formel (III) kann variieren. Vorzugsweise liegt das Verhältnis von Brønstedt-Säure zum Amin der Formel (III) im Bereich von etwa 10 : 0,6 bis etwa 1: 1,5, insbesondere von etwa 5: 0,9 bis 1: 1,2, speziell von etwa 2:1 bis etwa 1: 1,1.

Dabei ist es möglich, sowohl organische als auch anorganische Brønstedt-Säuren zu verwenden. Vorzugsweise werden anorganische Säuren, beispielsweise Phosphorsäure (H₃PO₄), Schwefelsäure (H₂SO₄), Salzsäure (HCl), Bromwasserstoffsäure (HBr), Flusssäure (HF) oder Kaliumhydrogensulfat (KHSO₄), verwendet. Die einzelnen Säuren können dabei sowohl in wasserfreier Form als auch in wasserhaltiger Form, beispielsweise als 85%ige Phosphorsäure oder 37%ige Salzsäure, d.h. insbesondere in Formen, in welchen die Säuren kommerziell erhältlich sind, verwendet werden. Beispiele für geeignete organische Säuren sind Trifluoressigsäure, Essigsäure, Methansulfonsäure und p-Toluolsulfonsäure. Von den zuvor genannten Säuren sind insbesondere Phosphorsäure, Schwefelsäure, Kaliumhydrogensulfat und Trifluoressigsäure bevorzugt.

Die Umsetzung zur Herstellung der Verbindungen der Formel (I) kann im Allgemeinen im Vakuum, bei Normaldruck oder unter Überdruck durchgeführt werden. Die angewendeten Temperaturen können ebenfalls, in Abhängigkeit der verwendeten Substrate, variieren und sind für den Fachmann durch Routineversuche leicht zu ermitteln. Beispielsweise kann die Umsetzung zur Herstellung der Verbindungen der Formel (I) bei einer Temperatur von 20 bis 200 °C, vorzugsweise 20 bis 150 °C, durchgeführt werden.

Die Stöchiometrie der eingesetzten Ausgangsverbindungen der Formeln (II) und (III) kann in weiten Bereichen variieren und unterliegt im Allgemeinen keiner besonderen Beschränkung. Geeignete Stöchiometrien der eingesetzten Ausgangsverbindungen der Formeln (II) und (III) können von dem Fachmann leicht durch Routineversuche ermittelt werden. So kann das molare Verhältnis der Verbindung der allgemeinen Formel (II) zu dem eingesetzten Amin der allgemeinen FFormel (III) beispielsweise 0,5 bis 10, insbesondere 1 bis 6, speziell 1,05 bis 2, betragen. Der Einsatz größerer Mengen an Verbindung der Formel (III) ist grundsätzlich möglich, jedoch aus wirtschaftlichen Gründen nachteilig.

Zum Ende der Reaktion kann das Reaktionswasser durch Destillation eines Teils des Lösungsmittel als Azeotrop entfernt werden. Bei hochsiedenden Lösungsmittel kann dies im Vakuum erfolgen. Durch diesen Vorgang erreicht man im Allgemeinen einen quantitativen Umsatz.

Falls die Umsetzung in einem Lösungsmittel durchgeführt wird, kann das Lösungsmittel nach Reaktionsende durch Abdestillieren entfernt werden. Dieses kann unter Normaldruck oder erniedrigtem Druck bei Raumtemperatur oder erhöhten Temperaturen erfolgen.

Die Isolierung der gewünschten Verbindungen der Formel (I) kann beispielsweise auch durch Kristallisation erfolgen.

Die vorliegende Erfindung wird anhand der nachfolgenden Beispiele näher erläutert, wobei die Beispiele nicht in die Erfindung einschränkende Weise zu interpretieren sind.

### Herstellungsbeispiele

### Beispiel 1: Herstellung von 4-[[(6-Chlorpyridin-3-yl)methyl](2,2-difluorethyl)amino]furan-2(5H)-on

Zu einer Suspension von 46 g 2-Ethoxy-4,5-dihydro-4-oxo-furan-3-carbonsäureethylester und 39,3 g N-[(6-Chlorpyridin-3-yl)methyl]-2,2-difluorethylamin in 705 ml Butyronitril wurden bei Raumtemperatur 31,2 g Kaliumhydrogensulfat zugesetzt. Die Mischung wurde 5 Stunden auf Rückfluß erhitzt. Anschließend wurde auf Raumtemperatur abgekühlt und mit 890 ml Wasser gewaschen. Das Lösungsmittel wurde im Vakuum entfernt. Man erhielt 51 g 4-[[(6-Chlorpyridin-3-yl)methyl](2,2-difluorethyl)amino]furan-2(5H)-on (dies entspricht 93 % Ausbeute).

¹H-NMR (CDCl₃, 298K) δ: 3,53 (td, 2H), 4,52 (s, 2H), 4,82 (s, 2H), 4,83 (s, 1H), 5,96 (tt, 1H), 7,37 (d, 1H), 7,55 (dd, 1H), 8,27(d, 1H)

### Beispiel 2: Herstellung von 2-Isopropoxy-4,5-dihydro-4-oxo-furan-3-carbonsäureisopropylester

20 g Malonsäurediisopropylester wurden in 173 g Toluol vorgelegt. Anschließend wurden bei Raumtemperatur 12 g Kalium-tert.-butylat in Portionen zugesetzt. Nach 2 Stunden wurde das Lösungsmittel im Vakuum entfernt und wieder mit 173 g Toluol versetzt. Bei 0°C wurden 6 g Chloracetylchlorid zugetropft und die Reaktionsmischung anschließend bei Raumtemperatur gerührt. Anschließend wurden 50 g Eis und 100 ml Wasser zugesetzt und 10 Minuten gerührt. Die organische Phase wurde abgetrennt und über Magnesiumsulfat getrocknet. Nach Entfernung des Lösungsmittels erhält man ein 1:1 Gemisch aus 2-Isopropoxy-4,5-dihydro-4-oxo-furan-3-carbonsäüreisopropylester (Ausbeute 45 %) und dem Malonsäurediisopropylester. Das Gemisch konnte so in die nächste Stufe eingesetzt werden. Mit Hilfe von Säulenchromatographie wurde der 2-Isopropoxy-4,5-dihydro-4-oxo-furan-3-carbonsäureisopropylester isoliert.

¹H-NMR (CDCl₃, 298K) δ: 1,3 d (6H), 1,50 d (6 H), 4,63 s (2 H), 5,14 m (1H), 5,29 m (1H), 8,56 s (1H), 8,75 d(1H)

### Beispiel 3: Herstellung von 4-[[(6-Chlorpyridin-3-yl)methyl](2,2-difluorethyl)amino]furan-2(5H)-on

Zu einer Suspension von 1,0 g 2-Isopropoxy-4,5-dihydro-4-oxo-furan-3-carbonsäureisopropylester und 0,85 g N-[(6-Chlorpyridin-3-yl)methyl]-2,2-difluorethylamin in 15,8 g Butyronitril wurden bei Raumtemperatur 0,6 g Kaliumhydrogensulfat zugesetzt. Die Mischung wurde 5 Stunden auf Rückfluss erhitzt. Anschließend wurde auf Raumtemperatur abgekühlt und mit 10 ml Wasser gewaschen. Das Lösungsmittel wurde im Vakuum entfernt. Man erhielt 1 g 4-[[(6-Chlorpyridin-3-yl)methyl](2,2-difluorethyl)amino]furan-2(5H)-on (dies entspricht 95 % Ausbeute).

¹H-NMR (CDCl₃, 298K) δ: 3,53 (td, 2H), 4,52 (s, 2H), 4,82 (s, 2H), 4,83 (s, 1H), 5,96 (tt, 1H), 7,37 (d, 1H), 7,55 (dd, 1H), 8,27(d, 1H)

## Patentansprüche

1. Verfahren zum Herstellen von Verbindungen der Formel (I) 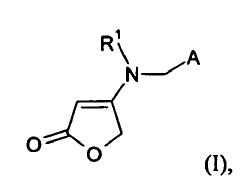 **dadurch gekennzeichnet, dass** Verbindungen der Formel (II) 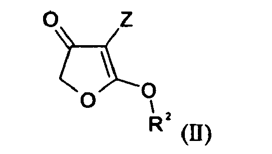 mit Aminen der Formel (III) 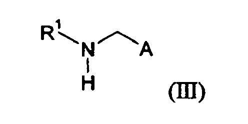 in Gegenwart einer Brønstedt-Säure umsetzt werden, wobei
R¹ für Wasserstoff, Alkyl, Halogenalkyl, Alkenyl, Halogenalkenyl, Alkinyl, Cycloalkyl, Cycloalkylalkyl, Halogencycloalkyl, Alkoxy, Alkyloxyalkyl, Halogencycloalkylalkyl oder Arylalkyl steht;
R² für Alkyl, Halogenalkyl, Alkenyl, Halogenalkenyl, Alkinyl, Cycloalkyl, Cycloalkylalkyl, Halogencycloalkyl, Alkyloxyalkyl, Halogencycloalkylalkyl, Aryl oder Arylalkyl steht;
Z ausgewählt ist aus den chemischen Gruppierungen -(C=O)OR³ und - (C=O)NR¹CH₂A, worin R³ ausgewählt ist aus C₁₋₁₂-Alkyl, C₃₋₈-Cycloalkyl, C₂₋₁₂ Alkenyl, C₂₋₁₂-Alkinyl, C₆₋₈-Aryl, C₇₋₁₉-Arylalkyl oder C₇₋₁₉-Alkylaryl, die jeweils mit einer oder mehreren Resten substituiert sein können, die ausgewählt sind unter -R', -OR', -SR', -NR'₂, -SiR'₃, -COOR', -(C=O)R', -CN and -CONR₂', wobei R' für Wasserstoff oder C₁₋₁₂-Alkyl steht;
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlorpyrazin-5-yl oder für 1,3-Thiazol-5-yl, welches gegebenenfalls in 2-Position substituiert ist durch Chlor oder Methyl, oder
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano, Nitro, C₁-C₄-Alkyl, welches gegebenenfalls durch Fluor und/oder Chlor substituiert ist, C₁-C₃-Alkylthio, welches gegebenenfalls durch Fluor und/oder Chlor substituiert ist, oder C₁-C₃-Alkylsulfonyl, welches gegebenenfalls durch Fluor und/oder Chlor substituiert ist, substituiert ist, oder für 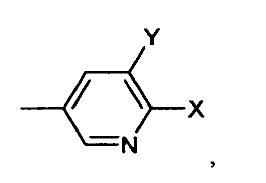 wobei
X für Halogen, Alkyl oder Halogenalkyl steht und
Y für Halogen, Alkyl, Halogenalkyl, Halogenalkoxy, Azido oder Cyan steht.

2. Verfahren nach Anspruch 1, **dadurch gekennzeichnet, dass** die Brønstedt-Säure ausgewählt ist unter H₃PO₄, H₂SO₄, HCl, HBr, HF, KHSO₄, Trifluoressigsäure, Essigsäure, Methansulfonsäure und p-Toluolsulfonsäure ist.

3. Verfahren nach Anspruch 1 oder 2, wobei
R¹ für Wasserstoff, C₁₋₁₂-Alkyl, C₁₋₁₂-Halogenalkyl, C₂₋₁₂-Alkenyl, C₂₋₁₂-Halogenalkenyl, C₂₋₁₂-Alkinyl, C₃₋₈-Cycloalkyl, C₃₋₈-CycloalkylC₁₋₁₂-alkyl, C₃₋₈-Halogencycloalkyl, C₁₋₁₂-Alkoxy, C₁₋₁₂-Alkyloxyalkyl, C₃₋₈-HalogencycloalkylC₁₋₁₂-alkyl oder C₆₋₁₄ArylC₁₋₁₂-alkyl steht;
R² für C₁₋₁₂-Alkyl, C₁₋₁₂-Halogenalkyl, C₂₋₁₂Alkenyl, C₂₋₁₂-Halogenalkenyl, C₂₋₁₂-Alkinyl, C₃₋₈-Cycloalkyl, C₃₋₈-CycloalkylC₁₋₁₂-alkyl, C₃₋₈-Halogencycloalkyl, C₁₋₁₂-Alkoxy, C₁₋₁₂-Alkyloxyalkyl, C₃₋₈-HalogencycloalkylC₁₋₁₂-alkyl oder C₆₋₁₄-ArylC₁₋₁₂-alkyl steht;
Z ausgewählt ist aus den chemischen Gruppierungen -(C=O)OR³ und - (C=O)NR¹CH₂A, worin R³ ausgewählt ist aus C₁₋₁₂-Alkyl, C₃₋₈-Cycloalkyl, C₂₋₁₂-Alkenyl, C₂₋₁₂-Alkinyl, C₆₋₈-Aryl, C₇₋₁₉-Arylalkyl oder C₇₋₁₉-Alkylaryl, die jeweils mit einer oder mehreren Resten substituiert sein können, die ausgewählt sind unter -R', -OR', -SR', -NR'₂, -SiR'₃, -COOR', -(C=O)R', -CN and -CONR₂', wobei R' für Wasserstoff oder C₁₋₁₂-Alkyl steht;
A für Pyrid-2-yl oder Pyrid-4-yl steht oder für Pyrid-3-yl, welches gegebenenfalls in 6-Position substituiert ist durch Fluor, Chlor, Brom, Methyl, Trifluormethyl oder Trifluormethoxy oder für Pyridazin-3-yl, welches gegebenenfalls in 6-Position substituiert ist durch Chlor oder Methyl oder für Pyrazin-3-yl oder für 2-Chlorpyrazin-5-yl oder für 1,3-Thiazol-5-yl, welches gegebenenfalls in 2-Position substituiert ist durch Chlor oder Methyl, oder
für Pyrimidinyl, Pyrazolyl, Thiophenyl, Oxazolyl, Isoxazolyl, 1,2,4-Oxadiazolyl, Isothiazolyl, 1,2,4-Triazolyl oder 1,2,5-Thiadiazolyl, welcher gegebenenfalls durch Fluor, Chlor, Brom, Cyano, Nitro, C₁-C₄-Alkyl, welches gegebenenfalls durch Fluor und/oder Chlor substituiert ist, C₁-C₃-Alkylthio, welches gegebenenfalls durch Fluor und/oder Chlor substituiert ist, oder C₁-C₃-Alkylsulfonyl, welches gegebenenfalls durch Fluor und/oder Chlor substituiert ist, substituiert ist, oder für 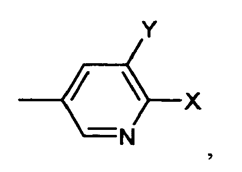 wobei
X für Chlor, Brom, Iod, Fluor, C₁₋₁₂-Alkyl oder C₁₋₁₂-Halogenalkyl steht und
Y für Chlor, Brom, Iod, Fluor, C₁₋₁₂-Alkyl oder C₁₋₁₂-Halogenalkyl, C₁₋₁₂-Halogenalkoxy, Azido oder Cyan steht.

4. Verfahren nach Anspruch 1 oder 2, wobei
A für 6-Fluor-pyrid-3-yl, 6-Chlor-pyrid-3-yl, 6-Brompyrid-3-yl, 6-Chlor-1,4-pyridazin-3-yl, 2-Chlor-1,3-thiazol-5-yl, 2-Chlor-pyrimidin-5-yl, 5-Fluor-6-chlorpyrid-3-yl, 5,6-Dichlor-pyrid-3-yl, 5-Brom-6-chlor-pyrid-3-yl, 5-Fluor-6-brompyrid-3-yl, 5-Chlor-6-brom-pyrid-3-yl, 5,6-Dibrom-pyrid-3-yl, 5-Methyl-6-chlorpyrid-3-yl, 5-Chlor-6-iod-pyrid-3-yl oder 5-Difluormethyl-6-chlor-pyrid-3-yl steht;
R¹ zur Methyl, Ethyl, n-Propyl, iso-Propyl, Vinyl, Allyl, Propargyl, Cyclopropyl, Alkoxyalkyl, 2-Fluor-ethyl, 2,2-Difluor-ethyl und 2-Fluor-cyclopropyl steht;
Z für eine chemische Gruppierung -(C=O)OR³ steht, worin R³ für Methyl, Ethyl, n-Propyl, iso-Propyl oder Butyl steht; und
R² für C₁-C₁₂-Alkyl steht.

5. Verfahren nach Anspruch 1 oder 2, wobei
A für 6-Chlor-pyrid-3-yl, 6-Brom-pyrid-3-yl, 6-Chlor-1,4-pyridazin-3-yl, 2-Chlor-1,3-thiazol-5-yl, 5-Fluor-6-chlor-pyrid-3-yl oder 5-Fluor-6-brom-pyrid-3-yl steht;
R¹ für Methyl, Ethyl, n-Propyl, iso-Propyl, n-Prop-2-enyl, n-Prop-2-inyl, Cyclopropyl, Methoxyethyl, 2-Fluorethyl oder 2,2-Difluor-ethyl steht;
Z für -CO₂CH₂CH₃ oder -CO₂CH₃; und
R² für Ethyl, Isopropyl oder Butyl steht.

6. Verfahren nach einem der Ansprüche 1 bis 5, **dadurch gekennzeichnet, dass** die Umsetzung in einem Lösungsmittel durchgeführt wird, das ausgewählt unter Dioxan, Butyronitril, Propionitril, Acetonitril, DME, Toluol, Methyl-THF, Dichlorbenzol, Chlorbenzol, n-Heptan, iso-Butanol, n-Butanol, Ethanol, Methyl-tert.-butylether, Isopropylethylether und Mischungen davon.

7. Verfahren nach einem der Ansprüche 1 bis 6, **dadurch gekennzeichnet, dass** die Umsetzung in einem Temperaturbereich von 20 °C bis 150 °C durchgeführt wird.

8. Verfahren nach einem der Ansprüche 1 bis 7, **dadurch gekennzeichnet, dass** das molare Verhältnis der Verbindung der Formel (II) zur Verbindung der Formel (III) 1 bis 6 beträgt.

9. Verbindungen der Formel (II) 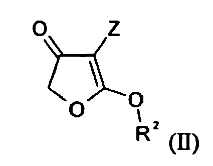 in welcher die Reste Z und R² wie in einem der Ansprüche 1, 3 oder 4 definiert sind, mit der Maßgabe, dass R² nicht für Ethyl oder Phenyl steht.

## Claims

1. Process for preparing compounds of the formula (I) 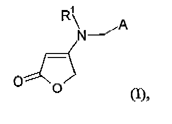 **characterized in that** compounds of the formula (II) 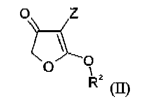 are reacted with amines of the formula (III) 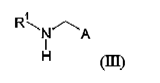 in the presence af a Brønsted acid, where
R¹ is hydrogen, alkyl, haloalkyl, alkenyl, haloalkenyl, alkynyl, cycloalkyl, cycloalkylalkyl, halocycloalkyl, alkoxy, alkyloxyalkyl halocycloalkylalkyl or arylalkyl;
R² is alkyl, haloalkyl, alkenyl, haloalkenyl, alkynyl, cycloalkyl, cycloalkylalkyl, halocycloalkyl, alkyloxyalkyl, halocycloalkylalkyl, aryl or arylalkyl;
Z is selected from the chemical moieties -(C=O)OR³ and -(C=O)NR¹CH₂A, in which R³ is selected from C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl or C₇₋₁₉-alkylaryl, each of which may be substituted by one or more radicals selected from -R', -OR', -SR', -NR'₂, -SiR'₃, -COOR', - (C=O)R', -CN and -CONR₂', where R' is hydrogen or C₁₋₁₂-alkyl;
A is pyrid-2-yl or pyrid-4-yl, or is pyrid-3-yl which is optionally 6-substituted by fluorine, chlorine, bromine, methyl, trifluoromethyl or trifluoromethoxy, or is pyridazin-3-yl which is optionally 6-substituted by chlorine or methyl, or is pyrazin-3-yl or is 2-chloropyrazin-5-yl or is 1,3-thiazol-5-yl which is optionally 2-substituted by chlorine or methyl, or
is pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by fluorine, chlorine, bromine, cyano, nitro, C₁-C₄-alkyl which is optionally substituted by fluorine and/or chlorine, C₁-C₃-alkylthio which is optionally substituted by fluorine and/or chlorine, or C₁-C₃-alkylsulphonyl which is optionally substituted by fluorine and/or chlorine, or is 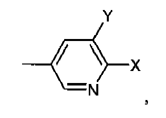 where
X is halogen, alkyl or haloalkyl and
Y is halogen, alkyl haloalkyl, haloalkoxy, azido or cyano.

2. Process according to Claim 1, **characterized in that** the Brønsted acid is selected from H₃PO₄, H₂SO₄, HCl, HBr, HF, KHSO₄, trifluoroacetic acid, acetic acid, methanesulphonic acid and p-toluenesulphonic acid.

3. Process according to Claim 1 or 2, where
R¹ is hydrogen, C₁₋₁₂-alkyl, C₁₋₁₂-haloalkyl, C₂₋₁₂-alkenyl, C₂₋₁₂-haloakenyl, C₂₋₁₂-alkynyl, C₃₋₈-cycloalkyl, C₃₋₈-cycloalkylC₁₋₁₂-alkyl, C₃₋₈-halocycloalkyl, C₁₋₁₂-alkoxy, C₁₋₁₂-alkyloxyalkyl, C₃₋₈-halocycloalkylC₁₋₁₂-alkyl or C₆₋₁₄-arylC₁₋₁₂-alkyl;
R² is C₁₋₁₂-alkyl, C₁₋₁₂-haloalkyl, C₂₋₁₂-alkenyl, C₂₋₁₂-haloalkenyl, C₂₋₁₂-alkynyl, C₃₋₈-cycloalkyl, C₃₋₈-cycloalkylC₁₋₁₂-alkyl, C₃₋₈-balocycloalkyl, C₁₋₁₂-alkoxy, C₁₋₁₂-akyloxyalkyl, C₃₋₈-halocycloalkylC₁₋₁₂-alkyl or C₆₋₁₄-ArylC₁₋₁₂-alkyl;
Z is selected from the chemical moieties -(C=O)OR³ and -(C=O)NR¹CH₂A, in which R³ is selected from C₁₋₁₂-alkyl, C₃₋₈-cycloalkyl, C₂₋₁₂-alkenyl, C₂₋₁₂-alkynyl, C₆₋₈-aryl, C₇₋₁₉-arylalkyl or C₇₋₁₉-alkylaryl, each of which may be substituted by one or more radicals selected from -R', -OR', -SR', -NR'₂, -SiR'₃, -COOR', - (C=O)R', -CN and -CONR₂', where R' is hydrogen or C₁₋₁₂-alkyl;
A is pyrid-2-yl or pyrid-4-yl, or is pyrid-3-yl which is optionally 6-substituted by fluorine, chlorine, bromine, methyl, trifluoromethyl or trifluoromethoxy, or is pyridazin-3-yl which is optionally 6-substituted by chlorine or methyl, or is pyrazin-3-yl or is 2-chloropyrazin-5-yl or is 1,3-thiazol-5-yl which is optionally 2-substituted by chlorine or methyl, or
is pyrimidinyl, pyrazolyl, thiophenyl, oxazolyl, isoxazolyl, 1,2,4-oxadiazolyl, isothiazolyl, 1,2,4-triazolyl or 1,2,5-thiadiazolyl, which is optionally substituted by fluorine, chlorine, bromine, cyano, nitro, C₁-C₄-alkyl which is optionally substituted by fluorine and/or chlorine, C₁-C₃-alkylthio which is optionally substituted by fluorine and/or chlorine, or C₁₋C₃-alkylsulphonyl which is optionally substituted by fluorine and/or chlorine, or is 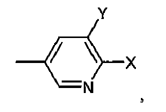 where
X is chlorine, bromine, iodine, fluorine, C₁₋₁₂-alkyl or C₁₋₁₂-haloalkyl and
Y is chlorine, bromine, iodine, fluorine, C₁₋₁₂-alkyl or C₁₋₁₂-haloalkyl, C₁₋₁₂-haloalkoxy, azido or cyano.

4. Process according to Claim 1 or 2, where
A is 6-fluoropyrid-3-yl, 6-chloropyrid-3-yl, 6-bromopyrid-3-yl, chloro-1,4-pyridazin-3-yl, 2-chloro-1,3-thiazol-5-yl, 2-chloropyrimidin-5-yl, 5-fluoro-6-chloropyrid-3-yl, 5,6-dichloropyrid-3-yl, 5-bromo-6-chloropyrid-3-yl, 5-fluoro-6-bromopyrid-3-yl, 5-chloro-6-bromopyrid-3-yl, 5,6-dibromopyrid-3-yl, 5-methyl-6-chloropyrid-3-yl, 5-chloro-6-iodopyrid-3-yl or 5-difluoromethyl-6-chloropyrid-3-yl;
R¹ is methyl, ethyl, n-propyl, isopropyl, vinyl, allyl, propargyl, cyclopropyl, alkoxyalkyl, 2-fluoroethyl, 2,2-difluoroethyl and 2-fluorocyclopropyl;
Z is a chemical moiety -(C=O)OR³ in which R³ is methyl, ethyl, n-propyl, isopropyl or butyl; and
R² is C₁₋C₁₂-alkyl.

5. Process according to Claim 1 or 2, where
A is 6-chloropyrid-3-yl, 6-bromopyrid-3-yl, 6-chloro-1,4-pyridazin-3-yl, 2-chloro-1,3-thiazol-5-yl, 5-fluoro-6-chloropyrid-3-yl or 5-fluoro-6-bromopyrid-3-yl;
R¹ is methyl, ethyl, n-propyl, isopropyl, n-prop-2-enyl, n-prop-2-ynyl, cyclopropyl, methoxyathyl, 2-fluoroethyl or 2,2-difluoroethyl;
Z is -CO₂CH₂CH₃ or -CO₂CH₃; and
R² is ethyl, isopropyl or butyl.

6. Process according to any of Claims 1 to 5, **characterized in that** the reaction is performed in a solvent which is selected from dioxane, butyronitrile, propionitrile, acetonitrile, DME, toluene, methyl-THF, dichlorobenzene, chlorobenzene, n-heptane, isobutanol, n-butanol, ethanol, methyl tert-butyl ether, isopropyl ethyl ether and mixtures thereof.

7. Process according to any of Claims 1 to 6, **characterized in that** the reaction is performed in a temperature range from 20°C to 150°C.

8. Process according to any of Claims 1 to 7, **characterized in that** the molar ratio of the compound of the formula (II) to the compound of the formula (III) is 1 to 6.

9. Compounds of the formula (II) 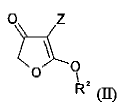 in which the Z and R² radicals are each as defined in any of Claims 1, 3 and 4, with the proviso that R² is not ethyl or phenyl.

## Revendications

1. Procédé pour la préparation de composés de formule (I) 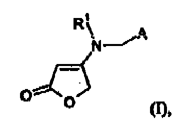 **caractérisée en ce qu'**on fait réagir des composés de formule (II) 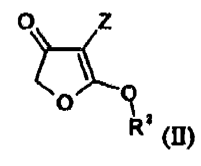 avec des amines de formule (III) 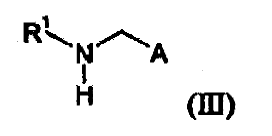 en présence d'un acide de Brønsted, où
R¹ représente un atome d'hydrogène, un groupe alkyle, halogénoalkyle, alcényle, halogénoalcényle, alcynyle, cycloalkyle, cycloalkylalkyle, halogénocycloalkyle, alcoxy, alkyloxyalkyle, halogénocycloalkylalkyle ou arylalkyle ;
R² représente un groupe alkyle, halogénoalkyle, alcényle, halogénoalcényle, alcynyle, cycloalkyle, cycloalkylalkyle, halogénocycloalkyle, alkyloxyalkyle, halogénocycloalkylalkyle, aryle ou arylalkyle ;
Z est choisi parmi les groupements -(C-O)OR³ et - (C=O)NR¹CH₂A, où R³ est choisi parmi des groupes alkyle en C₁-C₁₂, cycloalkyle en C₃-C₈, alcényle en C₂-C₁₂, alcynyle en C₂-C₁₂, aryle en C₆-C₈, arylalkyle en C₇-C₁₉ ou alkylaryle en C₇-C₁₉, qui peuvent être substitués chacun par un ou plusieurs radicaux, qui sont choisis parmi -R', -OR' , -SR', -NR'₂, -SiR'₃ -COOR', -(C=O)R', -CN et -CONR₂', R' représentant un atome d'hydrogène ou un groupe alkyle en C₁-C₁₂ ;
A représente le groupe pyrid-2-yle ou pyrid-4-yle ou représente un groupe pyrid-3-yle qui est éventuellement substitué en position 6 par un atome de fluor, chlore, brome, par un groupe méthyle, trifluorométhyle ou trifluorométhoxy ou représente un groupe pyridazin-3-yle qui est éventuellement substitué en position 6 par un atome de chlore ou par un groupe méthyle ou représente un groupe pyrazin-3-yle ou 2-chloropyrazin-5-yle ou représente un groupe 1,3-thiazol-5-yle qui est éventuellement substitué en position 2 par un atome de chlore ou par un groupe méthyle, ou représente
un groupe pyrimidinyle, pyrazolyle, thiophényle, oxazolyle, isoxazolyle, 1,2,4-oxadiazolyle, isothiazolyle, 1,2,4-triazvlyle ou 1,2,5-thiadiazvlyle, qui est éventuellement substitué par le fluor, le chlore, le brome, cyano, nitro, alkyle en C₁-C₄, qui est éventuellement substitué par le fluor et/ou le chlore, alkyl(C₁-C₃)thio, qui est éventuellement substitué par le fluor et/ou le chlore, ou alkyl(C₁-C₃)sulfonyle, qui est éventuellement substitué par le fluor et/ou le chlore, ou représente 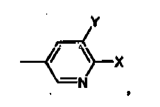 où
X représente un atome d'halogène, un groupe alkyle ou halogénoalkyle et
Y représente un atome d'halogène, un groupe alkyle, halogénoalkyle, halogénoalcoxy, azido ou cyano.

2. Procédé selon la revendication 1, **caractérisé en ce que** l'acide de Brønsted est choisi parmi H₃PO₄, H₂SO₄, HCl, HBr, HF, KHSO₄, l'acide trifluoroacétique, l'acide acétique, l'acide méthanesulfonique et l'acide p-toluénesulfonique.

3. Procédé selon la revendication 1 ou 2, dans lequel
R¹ représente un atome d'hydrogène, un groupe alkyle en C₁-C₁₂, halogénoalkyle en C₁-C₁₂, alcényle en C₂-C₁₂, halogénoalcényle en C₂-C₁₂, alcynyle en C₂-C₁₂, cycloalkyle en C₃-C₈, cycloalkyl(C₃-C₈)-alkyle(C₁-C₁₂), halogénocycloalkyle en C₃-C₈, alcoxy en C₁-C₁₂, alkyloxyalkyle (C₁-C₁₂) halogénocycloalkyl(C₃-C₈)alkyle(C₁-C₁₂) ou aryl(C₆-C₁₄)-alkyle(C₁-C₁₂) ;
R² représente un groupe alkyle en C₁-C₁₂, halogénoalkyle en C₁-C₁₂, alcényle en C₂-C₁₂, halogénoalcényle en C₂-C₁₂, alcynyle en C₂-C₁₂, cycloalkyle en C₃-C₈, cycloalkyl (C₃-C₈) alkyle (C₁-C₁₂), halogénocycloalkyle(C₃-C₈), alcoxy en C₁-C₁₂, alkyloxy(C₁-C₁₂)alkyle, halogénocycloalkyl(C₃-C₈)-alkyle(C₁-C₁₂) aryle ou aryl (C₆-C₁₄) alkyle (C₁-C₁₂) ;
Z est choisi parmi les groupements -(C=O)OR³ et - (C=O)NR¹CH₂A, où R³ est choisi parmi des groupes alkyle en C₁-C₁₂, cycloalkyle en C₃-C₈, alcényle en C₂-C₁₂, alcynyle en C₂-C₁₂, aryle en C₆-C₈, arylalkyle en C₇-C₁₉ ou alkylaryle en C₇-C₁₉, qui peuvent être substitués chacun par un ou plusieurs radicaux, qui sont choisis parmi -R', -OR', -SR', -NR'₂, -SiR'₃ -COOR', -(C=O)R', -CN et -CONR₂', R' représentant un atome d'hydrogène ou un groupe alkyle en C₁-C₁₂ ;
A représente le groupe pyrid-2-yle ou pyrid-4-yle ou représente un groupe pyrid-3-yle qui est éventuellement substitué en position 6 par un atome de fluor, chlore, brome, par un groupe méthyle, trifluorométhyle ou trifluorométhoxy ou représente un groupe pyridazin-3-yle qui est éventuellement substitué en position 6 par un atome de chlore ou par un groupe méthyle ou représente un groupe pyrazin-3-yle ou 2-chloropyrazin-5-yle ou représente un groupe 1,3-thiazol-5-yle qui est éventuellement substitué en position 2 par un atome de chlore ou par un groupe méthyle, ou représente
un groupe pyrimidinyle, pyrazolyle, thiophényle, oxazolyle, isoxazolyle, 1,2,4-oxadiazolyle, isothiazolyle, 1,2,4-triazolyle ou 1,2,5-thiadiazolyle, qui est éventuellement substitué par le fluor, le chlore, le brome, cyano, nitro, alkyle en C₁-C₄, qui est éventuellement substitué par le fluor et/ou le chlore, alkyl(C₁-C₃)thio, qui est éventuellement substitué par le fluor et/ou le chlore, ou alkyl(C₁-C₃)sulfonyle qui est éventuellement substitué par le fluor et/ou le chlore, ou représente 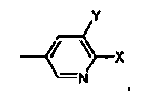 où
X représente un atome de chlore, brome, iode, fluor, un groupe alkyle en C₁-C₁₂ ou halogénoalkyle en C₁-C₁₂ et
Y représente un atome de chlore, brome, iode, fluor, un groupe alkyle en C₁-C₁₂ ou halogénoalkyle en C₁-C₁₂, halogénoalcoxy en C₁-C₁₂, azido ou cyano.

4. Procédé selon la revendication 1 ou 2, dans lequel
A représente le groupe 6-fluoro-pyrid-3-yle, 6-chloro-pyrid-3-yle, 6-bromo-pyrid-3-yle, 6-chloro-1,4-pyridazin-3-yle, 2-chloro-l,3-thiazol-5-yle, 2-chloro-pyrimidin-5-yle, 5-fluoro-6-chloro-pyrid-3-yle, 5,6-dichloro-pyrid-3-yle, 5-bromo-6-chloro-pyrid-3-yle, 5-fluoro-6-bromopyrid-3-yle, 5-chloro-6-bromo-pyrid-3-yle, 5,6-dibromo-pyrid-3-yle, 5-méthyl-6-chloro-pyrid-3-yle, 5-chloro-6-iodo-pyrid-3-yle ou 5-difluorométhyl-fi-chloro-pyrid-3-yle ;
R¹ représente un groupe méthyle, éthyle, n-propyle, isopropyle, vinyle, allyle, propargyle, cyclopropyle, alcoxyalkyle, 2-fluoro-éthyle, 2,2-difluoro-éthyle et 2-fluoro-cyclopropyle ;
Z représente un groupement chimique -(C=O)OR³, dans lequel R³ représente le groupe méthyle, éthyle, n-propyle, isopropyle ou butyle ; et
R² représente un groupe alkyle en C₁-C₁₂.

5. Procédé selon la revendication 1 ou 2, dans lequel
A représente le groupe 6-chloro-pyrid-3-yle, 6-bromo-pyrid-3-yle, 6-chloro-1,4-pyridazin-3-yle, 2-chloro-1,3-thiazol-5-yle, 5-fluoro-6-chloropyrid-3-yle ou 5-fluoro-6-bromo-pyrid-3-yle ;
R¹ représente le groupe méthyle, éthyle, n-propyle, isopropyle, n-prop-2-ényle, n-prop-2-ynyle, cyclopropyle, méthoxyéthyle, 2-fluoroéthyle ou 2,2-difluoroéthyle ;
Z représente -CO₂CH₂CH₃ ou -CO₂CH₃ ; et
R² représente le groupe éthyle, isopropyle ou butyle.

6. Procédé selon l'une quelconque des revendications 1 à 5, **caractérisé en ce qu'**on effectue la réaction dans un solvant qui est choisi parmi le dioxane, le butyronitrile, le propionitrile, l'acétonitrile, le DME, le toluène, le méthyl-THF, le dichlorobenzène, le chlorobenzène, le n-heptane, l'isobutanol, le n-butanol, l'éthanol, l'oxyde de méthyle et de tert-butyle, l'oxyde d'éthyle et d'isopropyle et des mélanges de ceux-ci.

7. Procédé selon l'une quelconque des revendications 1 à 6, **caractérisé en ce qu'**on effectue la réaction dans une plage de température de 20 °C à 150 °C.

8. Procédé selon l'une quelconque des revendications 1 à 7, **caractérisé en ce que** le rapport molaire du composé de formule (II) au composé de formule (III) vaut de 1 à 6.

9. Composés de formule (II) 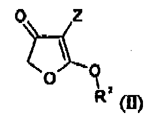 dans laquelle les radicaux Z et R² sont tels que définis dans l'une quelconque des revendications 1, 3 ou 4, étant entendu que R² ne représente pas le groupe éthyle ou phényle.
